(19) Europäisches Patentamt European Patent Office Office européen des brevets

(11) **EP 4 592 339 B1**

(12) **EUROPEAN PATENT SPECIFICATION**

(45) Date of publication and mention of the grant of the patent:
**17.06.2026 Bulletin 2026/25**

(21) Application number: **22963221.1**

(22) Date of filing: **02.11.2022**

(51) International Patent Classification (IPC):
**C08J 11/00** (2006.01)    **C08J 11/04** (2006.01)
**C08J 11/08** (2006.01)    **B01D 11/02** (2006.01)
**B29B 17/02** (2006.01)

(52) Cooperative Patent Classification (CPC):
(C-Sets available)
**C07C 29/82; B01D 9/00; B01D 11/0211;
B01D 11/0284; B01D 11/0288; B01D 11/0419;
B01D 11/0488; B01D 11/0492; C07C 67/03;
C07C 67/52; C08J 11/24;** C08J 2367/02;
Y02W 30/62        (Cont.)

(86) International application number:
**PCT/CN2022/129262**

(87) International publication number:
**WO 2024/087240 (02.05.2024 Gazette 2024/18)**

(54) **RECOVERY METHOD FOR FLAME-RETARDANT POLYESTER**

RÜCKGEWINNUNGSVERFAHREN FÜR FLAMMHEMMENDE POLYESTER

PROCÉDÉ DE RÉCUPÉRATION POUR POLYESTER IGNIFUGE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **26.10.2022 CN 202211319550**

(43) Date of publication of application:
**30.07.2025 Bulletin 2025/31**

(73) Proprietor: **National Industrial Innovation Center
of Polymer
Materials Co., Ltd
Guangzhou Hi-Tech Industrial Development
Zone
Guangzhou, Guangdong 510663 (CN)**

(72) Inventors:
• **WU, Bo
Guangzhou, Guangdong 510663 (CN)**

• **XIE, Xiaoqiong
Guangzhou, Guangdong 510663 (CN)**
• **LI, Jianjun
Guangzhou, Guangdong 510663 (CN)**
• **PANG, Chenghuan
Guangzhou, Guangdong 510663 (CN)**
• **WU, Hao
Guangzhou, Guangdong 510663 (CN)**

(74) Representative: **Petraz, Gilberto Luigi et al
GLP S.r.l.
Viale Europa Unita, 171
33100 Udine (IT)**

(56) References cited:
WO-A1-2014/099594    WO-A1-2019/174656
WO-A1-2022/033962    WO-A1-98/47952
CN-A- 107 739 434    CN-A- 114 031 756
GB-A- 762 690    JP-A- 2011 137 081

**(Cont. next page)**

(52) Cooperative Patent Classification (CPC): (Cont.)

C-Sets
**C07C 29/82, C07C 31/202;**
**C07C 29/82, C07C 31/207;**
**C07C 67/03, C07C 69/82;**
**C07C 67/52, C07C 69/82**

**Description**

## TECHNICAL FIELD

The present invention relates to the technical field of recovery of high-molecular compounds, and more specifically relates to a recovery method for a flame-retardant polyester.

## BACKGROUND

Global waste electrical and electronic equipment (WEEE) reaches 20-50 million tons every year, in which about 26% of WEEE plastics contain flame retardants (FRs). As most effective flame retardants at present, brominated flame retardants have been widely used for improving fireproof performance of plastic materials, and a global output thereof can reach 200,000 tons/year. Currently, the brominated flame retardants with large consumption at home and abroad mainly include tetrabromobisphenol A (TBBPA), decabromodiphenyl ether (DBDPO), hexabromocyclododecane (HBCD), decabromodiphenyl ethane (DBDPE), brominated epoxy resin (BEO), brominated polystyrene (BPS), etc. Bromine is a non-renewable resource. The price of the bromine in the global world has been constantly increasing since 2010, for which a main reason is that novel terminal bromine products are constantly iterated and updated. Meanwhile, due to mining of bromine resources in the world, the bromine resources are increasingly scarce, and mining difficulty is gradually increased, thus also exacerbating the increase of the bromine price. However, among all plastic wastes in the global world, only about 9% is recovered, 12% is incinerated, and 79% is piled up in a landfill or natural environment. Recycling the flame retardants and resins from flame-retardant plastics can not only decrease a landfill or incineration amount of the waste flame-retardant plastics in the environment to reduce the threat of pollution to the ecological environment, but also can make full use of waste products to reduce consumption of natural resources.

Currently, treatment methods for the flame-retardant plastics mainly include performing dehalogenation first and then performing mechanical recovery on dehalogenated plastics. Studies for debromination of waste electronic plastics are mainly focused on pyrolysis, catalytic pyrolysis, hydrothermal treatment and other technologies. According to these debromination technologies, high temperature decomposition of the flame retardants is usually required, but recycling values of the plastics are also destroyed during the high temperature decomposition. Compared with a dehalogenation technology with thermal treatment, the use of carbon dioxide supercritical extraction of the flame retardants or the use of solvent extraction of the flame retardants can ensure that the plastics themselves are not destroyed during the dehalogenation.

For example, according to a recovery method for a flame retardant in a waste circuit board disclosed in the prior art, the waste circuit board is first crushed, and then the flame retardant in the waste circuit board is recovered by using a $CO_2$ supercritical fluid as an extractant to obtain a triphenyl phosphate flame retardant. However, supercritical extraction has harsh operation conditions and high equipment requirements, is not suitable for large-scale industrial application, and is unable to recover a resin part. In addition, an anti-solvent precipitation method is adopted by Peng Shaohong, et al., to recover the flame retardants in the waste electronic plastics. However, according to the method, not only large amounts of solvents and anti-solvents are required to recover the flame retardants through dissolution, precipitation and extraction for several times, but also some polybrominated diphenyl ethers and unseparated fillers (antimony trioxide, glass fiber, carbon black, etc.) are remained in recycled plastics after solvent removal, thus affecting use of the recycled plastics.

WO 2022/033962 A1 discloses a method for the recovery of a flame-retardant from a polyester waste, which includes the extraction of the flame-retardant and the glycolysis of the polyester.

WO 2019/174656 A1 discloses a method for obtaining terephthalic acid from the depolymerization of waste polyethylene terephthalate in water by using microwave.

## SUMMARY

The purposes of the present invention are to overcome the defects and disadvantages that recycled plastics recovered from flame-retardant plastics have flame retardant residues and fillers are difficult to separate effectively, and to provide a recovery method for a flame-retardant polyester. By adopting separation and recovery methods corresponding to different flame-retardant polyesters and combining specific alcoholysis and extraction conditions with a separation sequence, a flame retardant in the flame-retardant polyester is efficiently recovered. Meanwhile, fillers and other impurities are separated from recovered resin, so that various components in the flame-retardant polyester are comprehensively recovered, and efficient cyclic utilization of the flame-retardant polyester is achieved.

The above purposes of the present invention are achieved through the following technical solutions.

A recovery method for a flame-retardant polyester includes determining the flame-retardant polyester to be a polyester A or a polyester B according to the type of a flame retardant; the polyester A is a polyester in which the flame retardant is one or more of brominated polystyrene, polydibromostyrene, decabromodiphenyl ethane, decabromodiphenyl ether, hexabromocyclododecane, and brominated epoxy; the polyester B is a polyester in which the flame retardant is one or more of a phosphate flame retardant, tetrabromobisphenol A, tetrabromobisphenol S, and brominated polycarbonate;

when the flame-retardant polyester is the polyester A, the method includes the following steps:

S1. subjecting the polyester A to crushing treatment;
S2. subjecting the polyester A crushed in the S1 to microwave alcoholysis, and performing separation and filtration to obtain a filtrate X and a filter residue a;
S3. subjecting the filtrate X in the S2 to microwave alcoholysis, then performing separation and purification to obtain a monomer or an oligomer and a filtrate Y, and purifying the filtrate Y to recover an alcohol substance;
S4. subjecting the filter residue a in the S2 to extraction with a targeted extractant, and performing separation and purification to obtain the flame retardant and an insoluble substance M;
wherein an alcoholysis agent used for the microwave alcoholysis in the S2 and the S3 is ethylene glycol or butanediol; the targeted extractant in the S4 is at least one of methanol, ethanol, isopropanol, acetone, chloroform, tetrahydrofuran, toluene, and limonene;

when the flame-retardant polyester is the polyester B, the method includes the following steps:

a1. subjecting the polyester B to crushing treatment;
a2. subjecting the polyester B crushed in the a1 to extraction treatment with a targeted extractant, and performing separation and purification to obtain the flame retardant and an insoluble substance N;
a3. subjecting the insoluble substance N in the a2 to microwave alcoholysis, and performing separation and filtration to obtain a filtrate E and a filter residue b;
a4. subjecting the filtrate E in the a3 to microwave alcoholysis, then performing separation and purification to obtain a monomer or an oligomer and a filtrate F, and purifying the filtrate F to recover an alcohol substance;
wherein the targeted extractant in the a2 is at least one of methanol, ethanol, isopropanol, acetone, chloroform, tetrahydrofuran, toluene, and limonene; and an alcoholysis agent used for the microwave alcoholysis in the a3 and the a4 is ethylene glycol or butanediol.

The inventor finds through a large number of studies that by selecting a small amount of a sample to determine the type of the flame retardant through a conventional component analysis method, then adopting the separation and recovery methods corresponding to the respective flame-retardant polyesters according to the type of the flame retardant in the flame-retardant polyester and combing specific microwave alcoholysis and extraction processes, the flame retardant in the flame-retardant polyester can be fully and efficiently recovered. Meanwhile, the filler and other impurities are separated from the recovered resin, so that the various components in the flame-retardant polyester are comprehensively recovered.

When the flame retardant in the flame-retardant polyester is a difficult-to-hydrolyze flame retardant (being one or more of the brominated polystyrene, the polydibromostyrene, the decabromodiphenyl ethane, the decabromodiphenyl ether, the hexabromocyclododecane, and the brominated epoxy), the flame retardant has limited dissolution in the targeted extractant, the flame retardant is difficult to extract directly from the flame-retardant polyester, and the flame retardant and other components can only be separated and recovered by adopting the recovery method of first releasing the flame retardant from the flame-retardant polyester through the microwave alcoholysis and then combing the specific targeted extractant for extraction.

When the flame retardant in the flame-retardant polyester is an easy-to-hydrolyze flame retardant (being one or more of the phosphate flame retardant, the tetrabromobisphenol A, the tetrabromobisphenol S, and the brominated polycarbonate), the flame retardant needs to be extracted and recovered from the flame-retardant polyester first, and then the microwave alcoholysis is performed to recover the resin. The reasons are that during recovery of the easy-to-hydrolyze flame retardant, when the microwave alcoholysis is performed first, the flame retardant is destructed during the microwave alcoholysis, which not only affects the quality of the recovered flame retardant, but also affects the recovery rate of the flame retardant.

In specific embodiments, when the alcohololysis agent in the present invention is the butanediol, a temperature of the microwave alcoholysis in the S2 and the S3 is 220°C - 230°C, and a duration of the microwave alcoholysis is 10 min - 30 min.

In specific embodiments, when the alcoholysis agent in the present invention is the ethylene glycol, a temperature of the microwave alcoholysis in the a3 and the a4 is 200°C - 210°C, and a duration of the microwave alcoholysis is 5 min - 10 min.

In specific embodiments, when the flame-retardant polyester is the polyester A, the insoluble substance M is dissolved with aqueous ammonia to separate and recover antimony trioxide; and when the flame-retardant polyester is the polyester B, the filter residue b in the step a3 is treated with a zinc chloride solution to recover antimony trioxide.

Specifically, a catalyst used for the microwave alcoholysis in the S2 and the S3 of the present invention is any one of zinc acetate, butyl titanate, a titanate nanotube, titanium glycolate, a 1,3-dimethylimidazolium tetrachloroferrate ionic liquid, a 1-allyl-3-methylimidazolium ionic liquid, and ferric chloride.

Specifically, a catalyst used for the microwave alcoholysis in the a3 and the a4 of the present invention is one or more of zinc acetate, butyl titanate, a titanate nanotube, titanium glycolate, a 1,3-dimethylimidazolium tetrachloroferrate ionic liquid, a 1-allyl-3-methylimidazolium ionic liquid, and ferric chloride.

Specifically, an addition amount of the catalyst of the present invention is 0.05%-1% of a mass of the flame-retardant polyester.

In specific embodiments, the extraction in the step S4 of the present invention is microwave extraction, an extraction temperature is 35°C - 160°C, and an extraction duration is 10 min - 120 min.

In specific embodiments, the extraction in the step a2 of the present invention is microwave extraction, an extraction temperature is 35°C - 160°C, and an extraction duration is 10 min - 120 min.

A microwave heating method is adopted in the present invention. Compared with other heating methods, microwave heating can achieve heating of the inside and outside of a material simultaneously and has a high and uniform heating speed, which not only greatly shortens the reaction time, but also can effectively reduce an impact on thermal stability of the bromine-containing flame retardant during the extraction. When the extraction temperature is too high, boiling is caused; and when the extraction temperature is too low, extraction efficiency is reduced. When the extraction duration is too long, the thermal stability of the recovered flame retardant is affected; and when the extraction duration is too short, the flame retardant in the polyester is difficult to fully recover.

Specifically, an average particle size of the polyester A after the crushing treatment in the S1 of the present invention is 2 mm - 10 mm; and an average particle size of the polyester B after the crushing treatment in the a1 is 0.02 mm - 1 mm. During practical production, no matter for polyester A particles or polyester B particles, the smaller average particle size is more conducive to subsequent treatment. However, when the particle size is too small, energy consumption is increased. Based on comprehensive consideration, application demands can be met when the average particle size of the flame-retardant polyester A particles is ≤10 mm and the average particle size of the flame-retardant polyester B particles is ≤1 mm.

Compared with the prior art, the present invention has the following beneficial effects.

According to the recovery method for the flame-retardant polyester provided in the present invention, by adopting the separation and recovery methods corresponding to the different flame-retardant polyesters and combining the specific alcoholysis and extraction conditions with the separation sequence, the flame retardant in the flame-retardant polyester is efficiently recovered. Meanwhile, the filler and other impurities are separated from the recovered resin, so that various components in the flame-retardant polyester are comprehensively recovered, and efficient cyclic utilization of the flame-retardant polyester is achieved. A recovery rate of the flame retardant reaches 80%-92%, and a recovery rate of the polyester resin reaches 80%-95%.

## BRIEF DESCRIPTION OF DRAWINGS

FIG. 1 shows a flow chart of recovering a waste flame-retardant polyester in Example 1;
FIG. 2 shows a flow chart of recovering a waste flame-retardant polyester in Example 6;
FIG. 3 shows an infrared spectrogram of waste flame-retardant PBT (a) and a microwave alcoholysis product (b) in Example 1; and
FIG. 4 shows an infrared spectrogram of a recovered brominated epoxy resin flame retardant (a) and a brominated epoxy original sample (b) in Example 1.

## DETAILED DESCRIPTION OF EMBODIMENTS

The present invention is further illustrated below in combination with specific embodiments, but the present invention is not limited to examples in any form. Unless otherwise specified, raw materials and reagents used in the examples of the present invention are conventionally purchased raw materials and reagents.

1. Main raw materials and reagents

A waste flame-retardant polyester 1 is flame-retardant PBT, which includes the following main components: PBT, brominated epoxy, EVA, antimony trioxide, a glass fiber, and other auxiliary agents.

A waste flame-retardant polyester 2 is flame-retardant PET, which includes the following main components: PET, brominated polystyrene, antimony trioxide, a glass fiber, and other auxiliary agents.

A waste flame-retardant polyester 3 is flame-retardant PBT/PET, which includes the following main components:

PBT/PET, tetrabromobisphenol A, antimony trioxide, a glass fiber, and other auxiliary agents.

A waste flame-retardant polyester 4 is flame-retardant PC, which includes the following main components: PC, triphenyl phosphate (BDP), antimony trioxide, a glass fiber, and other auxiliary agents.

A targeted extractant is methanol, ethanol, chloroform, tetrahydrofuran, or acetone.

An alcoholysis agent 1 is ethylene glycol, and an alcoholysis agent 2 is butanediol.

A catalyst 1 is zinc acetate, and a catalyst 2 is butyl titanate.

2. Performance tests

(1) Infrared spectrum test

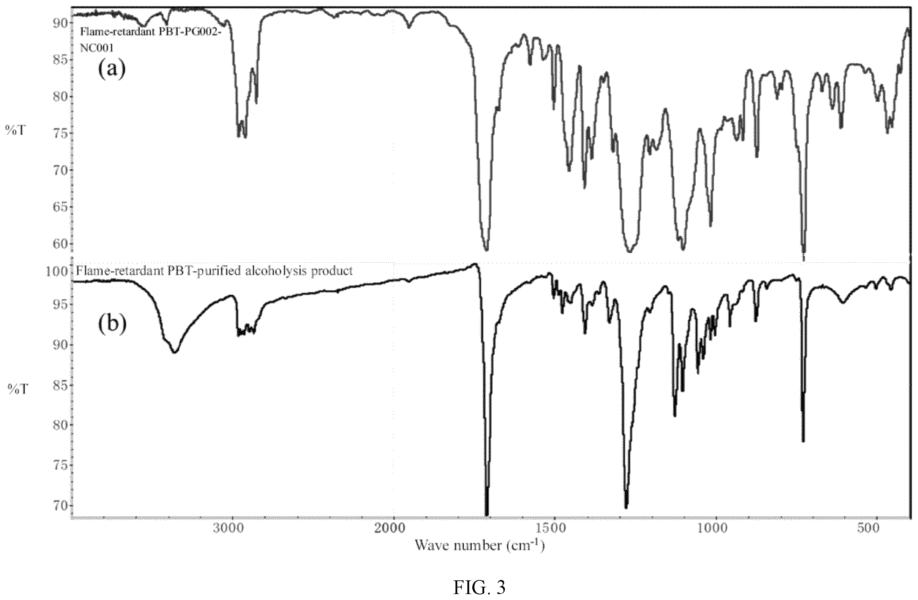

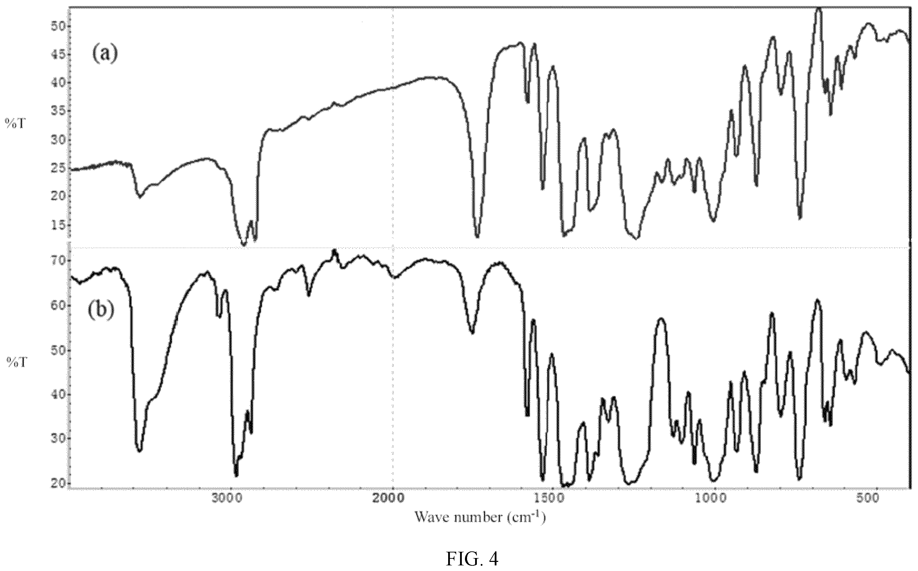

As can be seen from FIG. 3, a curve a represents an infrared spectrum of waste flame-retardant PBT, and an absorption peak at 1717.52 cm$^{-1}$ is a characteristic absorption peak of an ester group; and a curve b represents an infrared spectrum of a microwave alcoholysis product, and a strong and wide peak at 3370.45 cm$^{-1}$ is a stretching vibration absorption peak of a hydroxyl group (-OH). It can be inferred that the hydroxyl group is possible to exist in a depolymerization product of polyester. Meanwhile, a methylene group has an absorption peak at 2932.15 cm$^{-1}$ in the curve b, indicating that the methylene group exists in the microwave alcoholysis product; C-H on a benzene ring has a stretching vibration characteristic absorption peak at 2960.12 cm$^{-1}$, indicating that the microwave alcoholysis product contains the benzene ring; and a benzene ring para-substituent has a strong absorption peak at 750.24 cm$^{-1}$, indicating that the microwave alcoholysis product not only contains the benzene ring in structure, but also contains the benzene ring para-substituent. According to the curve a and the curve b in FIG. 3, it is fully indicated that a waste polyester is completely depolymerized into monomers. As can be seen from FIG. 4, a curve a represents a recovered brominated epoxy resin, and a curve b represents a brominated epoxy original sample, wherein in the curve a, -C-O-C- in an epoxy group has asymmetric stretching vibration peaks at 910.33 cm$^{-1}$, 870.55 cm$^{-1}$ and 797.99 cm$^{-1}$; a C=O ester group has a stretching vibration peak at 1738 cm$^{-1}$, and other absorption peaks are attributed as follows: a benzene ring has characteristic absorption peaks at 3081 cm$^{-1}$, 1583 cm$^{-1}$ and 1536 cm$^{-1}$; a methylene group has a characteristic absorption peak at 1467 cm$^{-1}$, and methylidyne in the epoxy group has characteristic absorption peaks at 2967.52 cm$^{-1}$ and 2929.25 cm$^{-1}$; an ether bond has antisymmetric stretching vibrations at 1065 cm$^{-1}$, 1127 cm$^{-1}$ and 1250 cm$^{-1}$; a methyl has a characteristic peak at 1386 cm$^{-1}$; and C-Br has characteristic absorption peaks at 641 cm$^{-1}$, 659 cm$^{-1}$ and 738 cm$^{-1}$, which basically and completely coincide with those of the brominated epoxy original sample.

(2) XRF test

The test is carried out by using a Gonio method with LiF 200 as a diffraction crystal, a collimator at 150 $\mu$m, a scintillation counter as a detector and Al (750 $\mu$m) as a filter. In the present invention, an external standard method is adopted for quantitative analysis of bromine. Bromine contents of a waste flame-retardant polyester, a recovered brominated epoxy flame retardant and a brominated epoxy original sample in Example 1 are shown in Table 1. It can be seen that the bromine content of the recovered brominated epoxy flame retardant is the same as the bromine content of the brominated epoxy original sample.

Table 1

| Item | Bromine content |
|---|---|
| Waste flame-retardant polyester | 4.2% |
| Recovered brominated epoxy flame retardant | 28% |
| Brominated epoxy original sample | 28% |

(3) TGA test

The TGA test is carried out in a temperature range of 30°C - 750°C at a heating rate of 10-20°C/min, a test atmosphere is nitrogen, and an initial decomposition temperature is analyzed and determined specifically by referring to the standard ISO 11358-1: 2014.

(4) Recovery rates of a flame retardant and a resin monomer

Flame retardant recovery rate = mass of recovered flame retardant/mass of flame retardant in flame-retardant polyester*100%;

a specific test method includes: weighing a small amount of the flame-retardant polyester to determine the type of the flame retardant contained through XRF analysis, and then determining a bromine content or phosphorus content in components of the flame retardant per unit according to the type of the flame retardant; then analyzing and determining a total bromine content or phosphorus content in the flame-retardant polyester, and dividing the total bromine content or phosphorus content by the bromine content or phosphorus content in the components of the flame retardant per unit to determine the mass of the flame retardant in the flame-retardant polyester; and directly weighing the mass of the recovered flame retardant, and obtaining the flame retardant recovery rate by calculation using the above formula.

Polyester monomer recovery rate=(mass of recovered monomer/relative molecular mass of monomer)/(mass of polyester in flame-retardant polyester/relative molecular mass of polyester repeat unit)* 100%;

wherein the mass of the polyester in the flame-retardant polyester = mass of the flame-retardant polyester - mass of the flame retardant in the flame-retardant polyester - mass of an insoluble substance, the mass of the flame retardant in the flame-retardant polyester is obtained according to the above calculation method, and the mass of the insoluble substance can be directly weighed.

Example 1

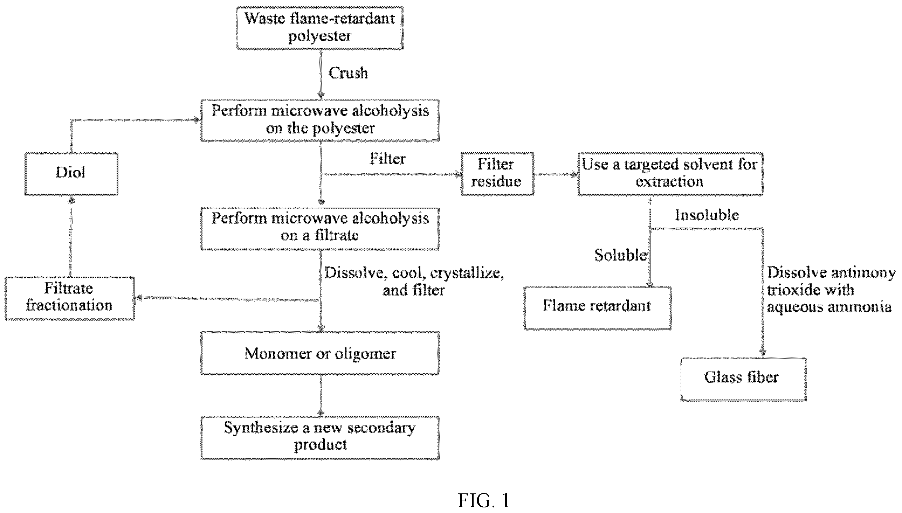

A recovery method for a flame-retardant polyester included the following steps (as shown in FIG. 1).
S1. A waste flame-retardant polyester 1 was subjected to crushing treatment to obtain particles with an average particle size of 10 mm.
S2. 150 parts of the crushed waste flame-retardant polyester 1 in the S1, 150 parts of butanediol and 0.3 part of zinc acetate were weighed in part by weight and added into a microwave reaction device for microwave alcoholysis at the condition of 220°C for 30 min, and separation and filtration were performed to obtain a filtrate X and a filter residue a.
S3. The filtrate X in the S2 was subjected to secondary microwave alcoholysis at the condition of 220°C for 30 min and then separated to obtain a clarified solution, the clarified solution was introduced into hot water (100°C) for repeated crystallization and purification to obtain hydroxybutyl terephthalate, and the remaining solution was subjected to azeotropic vacuum distillation to recover the butanediol.
S4. The filter residue a in the S2 was cleaned with ethanol, then tetrahydrofuran was added to remove EVA, filtration was performed, then a targeted extractant 2 (chloroform) was added into a filter cake for extraction at the condition of 50°C for 20 min, separation and purification were performed to obtain a flame retardant (brominated epoxy resin) and an insoluble substance M, the insoluble substance M was added into aqueous ammonia for dissolution to obtain antimony trioxide as a soluble substance and a glass fiber as an insoluble substance, and the glass fiber was washed with acetone and then recovered.

Example 2

A recovery method for a flame-retardant polyester included basically the same steps as those in Example 1, with the differences that a temperature of the microwave alcoholysis in the steps S2 and S3 was 230°C, and an alcoholysis duration was 10 min.

Example 3

A recovery method for a flame-retardant polyester included basically the same steps as those in Example 1, with the differences that a temperature of the microwave alcoholysis in the steps S2 and S3 was 210°C, and an alcoholysis duration was 150 min.

Example 4

A recovery method for a flame-retardant polyester included basically the same steps as those in Example 1, with the differences that a temperature of the microwave alcoholysis in the steps S2 and S3 was 215°C, and an alcoholysis duration was 60 min.

Example 5

A recovery method for a flame-retardant polyester included basically the same steps as those in Example 1, with the differences that an alcoholysis agent in the steps S2 and S3 was ethylene glycol, and the waste flame-retardant polyester in the step S1 was a waste flame-retardant polyester 2.

S1. The waste flame-retardant polyester 2 was subjected to crushing treatment to obtain particles with an average particle size of 10 mm.

S2. 150 parts of the crushed waste flame-retardant polyester 2 in the S1, 300 parts of the ethylene glycol and 0.3 part of zinc acetate were weighed in part by weight and added into a microwave reaction device for microwave alcoholysis at the condition of 195°C for 30 min, and separation and filtration were performed to obtain a filtrate X and a filter residue a.

S3. The filtrate X in the S2 was subjected to secondary microwave alcoholysis at the condition of 195°C for 30 min and then separated to obtain a clarified solution, the clarified solution was introduced into hot water (100°C) for repeated crystallization and purification to obtain hydroxyethyl terephthalate, and the remaining solution was subjected to azeotropic vacuum distillation to recover the ethylene glycol.

S4. The filter residue a in the S2 was cleaned with ethanol, then tetrahydrofuran was added to remove EVA, filtration was performed, then a targeted extractant 2 (chloroform) was added into a filter cake for extraction at the condition of 50°C for 20 min, separation and purification were performed to obtain a flame retardant (brominated epoxy resin) and an insoluble substance M, the insoluble substance M was added into aqueous ammonia for dissolution to obtain antimony trioxide as a soluble substance and a glass fiber as an insoluble substance, and the glass fiber was washed with acetone and then recovered.

Example 6

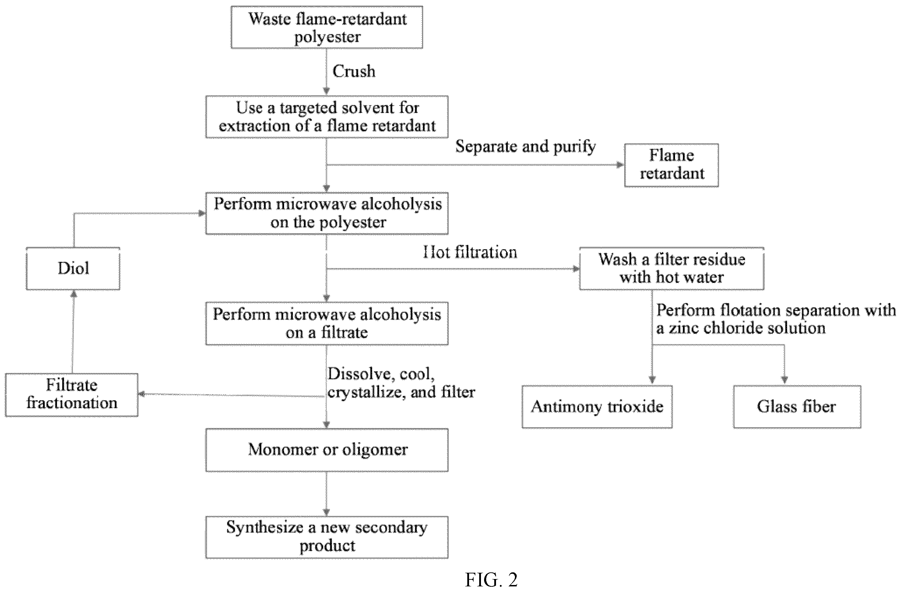

A recovery method for a flame-retardant polyester included the following steps (as shown in FIG. 2).

a1. A waste flame-retardant polyester 3 was subjected to crushing treatment to obtain particles with an average particle size of 1 mm.

a2. The crushed waste flame-retardant polyester 3 in the a1 was subjected to microwave alcoholysis with acetone as a targeted extractant at the condition of 50°C for 30 min, and separation and purification were performed to obtain a flame retardant (tetrabromobisphenol A) and an insoluble substance N.

a3. 150 parts of the insoluble substance N in the a2, 300 parts of ethylene glycol and 0.15 part of butyl titanate were weighed in part by weight and added into a microwave reaction device for microwave alcoholysis at the condition of 195°C for 20 min, and separation and filtration were performed to obtain a filtrate E and a filter residue b.

a4. The filtrate E in the a3 was subjected to secondary microwave alcoholysis at the condition of 195°C for 20 min and then separated to obtain a clarified solution, the clarified solution was introduced into hot water (100°C) for repeated crystallization and purification to obtain hydroxyethyl terephthalate, and the remaining solution was subjected to azeotropic vacuum distillation to recover the ethylene glycol; and meanwhile, the filter residue b in the S3 was added into a zinc chloride solution for flotation separation to obtain antimony trioxide and a glass fiber, and the glass fiber was washed with acetone and then recovered.

Example 7

A recovery method for a flame-retardant polyester included basically the same steps as those in Example 6, with the differences that a temperature of the microwave alcoholysis in the steps a3 and a4 was 210°C, and an alcoholysis duration was 5 min.

Example 8

A recovery method for a flame-retardant polyester included basically the same steps as those in Example 6, with the differences that a temperature of the microwave alcoholysis in the steps a3 and a4 was 190°C, and an alcoholysis duration was 150 min.

Example 9

A recovery method for a flame-retardant polyester included basically the same steps as those in Example 6, with the differences that a temperature of the microwave alcoholysis in the steps a3 and a4 was 200°C, and an alcoholysis duration was 10 min.

Example 10

A recovery method for a flame-retardant polyester included basically the same steps as those in Example 6, with the differences that the waste flame-retardant polyester in the step a1 was a waste flame-retardant polyester 4, and the targeted extractant in the step a2 was ethanol.

Comparative Example 1

A recovery method for a flame-retardant polyester included the following steps (as shown in FIG. 2).

a1. A waste flame-retardant polyester 1 was subjected to crushing treatment to obtain particles with an average particle size of 1 mm.
a2. The crushed waste flame-retardant polyester 1 in the a1 was subjected to microwave alcoholysis with chloroform as a targeted extractant at the condition of 50°C for 30 min, and separation and purification were performed to obtain a flame retardant (brominated epoxy resin) and an insoluble substance N.
a3. 150 parts of the insoluble substance N in the a2, 150 parts of butanediol and 0.15 part of butyl titanate were weighed in part by weight and added into a microwave reaction device for microwave alcoholysis at the condition of 220°C for 20 min, and separation and filtration were performed to obtain a filtrate E and a filter residue b.
a4. The filtrate E in the a3 was subjected to secondary microwave alcoholysis at the condition of 220°C for 20 min and then separated to obtain a clarified solution, the clarified solution was introduced into hot water (100°C) for repeated crystallization and purification to obtain dihydroxybutyl terephthalate, and the remaining solution was subjected to azeotropic vacuum distillation to recover the butanediol; and meanwhile, the filter residue b in the a3 was added into a zinc chloride solution for flotation separation to obtain antimony trioxide and a glass fiber, and the glass fiber was washed with acetone and then recovered.

Comparative Example 2

A recovery method for a flame-retardant polyester included the following steps (as shown in FIG. 1).
S1. A waste flame-retardant polyester 3 was subjected to crushing treatment to obtain particles with an average particle size of 10 mm.
S2. 150 parts of the crushed waste flame-retardant polyester 3 in the S1, 150 parts of ethylene glycol and 0.75 part of zinc acetate were weighed in part by weight and added into a microwave reaction device for microwave alcoholysis at the condition of 195°C for 30 min, and separation and filtration were performed to obtain a filtrate X and a filter residue a.
S3. The filtrate X in the S2 was subjected to secondary microwave alcoholysis at the condition of 195°C for 30 min and then separated to obtain a clarified solution, the clarified solution was introduced into hot water (100°C) for repeated crystallization and purification to obtain hydroxyethyl terephthalate, and the remaining solution was subjected to azeotropic vacuum distillation to recover the ethylene glycol.
S4. The filter residue a in the S2 was cleaned with ethanol, then a targeted extractant (chloroform) was added for extraction at the condition of 50°C for 20 min, separation and purification were performed to obtain a flame retardant (tetrabromobisphenol A) and an insoluble substance M, the insoluble substance M was added into aqueous ammonia for dissolution to obtain antimony trioxide as a soluble substance and a glass fiber as an insoluble substance, and the glass fiber was washed with acetone and then recovered.
Performance test results of products obtained according to the above recovery methods for the flame-retardant polyesters are shown in Table 2.

Table 2 Test Results of various examples and comparative examples

| Number | Flame retardant recovery rate | Initial decomposition temperature of a flame retardant | Polyester monomer recovery rate |
|---|---|---|---|
| Example 1 | 88% | 380.2°C | 90% |
| Example 2 | 89% | 380°C | 92% |
| Example 3 | 80% | 376°C | 80% |
| Example 4 | 86% | 380°C | 89% |
| Example 5 | 86% | 323°C | 95% |
| Example 6 | 87% | 410°C | 90% |
| Example 7 | 86% | 408°C | 91% |

(continued)

| Number | Flame retardant recovery rate | Initial decomposition temperature of a flame retardant | Polyester monomer recovery rate |
|---|---|---|---|
| Example 8 | 80% | 409°C | 81% |
| Example 9 | 88% | 408°C | 92% |
| Example 10 | 92% | 408°C | 90% |
| Comparative Example 1 | 78% | 380°C | 90% |
| Comparative Example 2 | 2% | 330°C | 85% |

**Claims**

1. A recovery method for a flame-retardant polyester, **characterized in that**

the flame-retardant polyester is determined to be a polyester A or a polyester B according to a type of a flame retardant; the polyester A is a polyester in which the flame retardant is one or more of brominated polystyrene, polydibromostyrene, decabromodiphenyl ethane, decabromodiphenyl ether, hexabromocyclododecane, and brominated epoxy; the polyester B is a polyester in which the flame retardant is one or more of a phosphate flame retardant, tetrabromobisphenol A, tetrabromobisphenol S, and brominated polycarbonate;
when the flame-retardant polyester is the polyester A, the method comprises the following steps:

S1, subjecting the polyester A to crushing treatment;
S2, subjecting the polyester A crushed in the S1 to microwave alcoholysis, and performing separation and filtration to obtain a filtrate X and a filter residue a;
S3, subjecting the filtrate X in the S2 to microwave alcoholysis, then performing separation and purification to obtain a monomer or an oligomer and a filtrate Y, and purifying the filtrate Y to recover an alcohol substance;
S4, subjecting the filter residue a in the S2 to extraction with a targeted extractant, and performing separation and purification to obtain the flame retardant and an insoluble substance M;
wherein an alcoholysis agent used for the microwave alcoholysis in the S2 and the S3 is ethylene glycol or butanediol; the targeted extractant in the S4 is at least one of methanol, ethanol, isopropanol, acetone, chloroform, tetrahydrofuran, toluene, and limonene;
when the flame-retardant polyester is the polyester B, the method comprises the following steps:

a1, subjecting the polyester B to crushing treatment;
a2, subjecting the polyester B crushed in the a1 to extraction with a targeted extractant, and performing separation and purification to obtain the flame retardant and an insoluble substance N;
a3, subjecting the insoluble substance N in the a2 to microwave alcoholysis, and performing separation and filtration to obtain a filtrate E and a filter residue b;
a4, subjecting the filtrate E in the a3 to microwave alcoholysis, then performing separation and purification to obtain a monomer or an oligomer and a filtrate F, and purifying the filtrate F to recover an alcohol substance;
wherein the targeted extractant in the a2 is at least one of methanol, ethanol, isopropanol, acetone, chloroform, tetrahydrofuran, toluene, and limonene; and an alcoholysis agent used for the microwave alcoholysis in the a3 and the a4 is ethylene glycol or butanediol.

2. The recovery method for a flame-retardant polyester according to claim 1, **characterized in that** when the alcohololysis agent is the butanediol, a temperature of the microwave alcoholysis in the S2 and the S3 is 220°C - 230°C, and a duration of the microwave alcoholysis is 10 min - 30 min.

3. The recovery method for a flame-retardant polyester according to claim 1, **characterized in that** when the alcoholysis agent is the ethylene glycol, a temperature of the microwave alcoholysis in the a3 and the a4 is 200°C - 210°C, and a duration of the microwave alcoholysis is 5 min - 10 min.

4. The recovery method for a flame-retardant polyester according to claim 1, **characterized in that** the insoluble substance M is dissolved with aqueous ammonia to separate and recover antimony trioxide.

**5.** The recovery method for a flame-retardant polyester according to claim 1, **characterized in that** a catalyst used for the microwave alcoholysis in the S2 and the S3 is one or more of zinc acetate, butyl titanate, a titanate nanotube, titanium glycolate, a 1,3-dimethylimidazolium tetrachloroferrate ionic liquid, a 1-allyl-3-methylimidazolium ionic liquid, and ferric chloride.

**6.** The recovery method for a flame-retardant polyester according to claim 1, **characterized in that** a catalyst used for the microwave alcoholysis in the a3 and the a4 is one or more of zinc acetate, butyl titanate, a titanate nanotube, titanium glycolate, a 1,3-dimethylimidazolium tetrachloroferrate ionic liquid, a 1-allyl-3-methylimidazolium ionic liquid, and ferric chloride.

**7.** The recovery method for a flame-retardant polyester according to claim 5 or 6, **characterized in that** an addition amount of the catalyst is 0.05% - 1% of a mass of the flame-retardant polyester.

**8.** The recovery method for a flame-retardant polyester according to claim 1, **characterized in that** the extraction in the S4 is microwave extraction, an extraction temperature is 35°C - 160°C, and an extraction duration is 10 min - 120 min.

**9.** The recovery method for a flame-retardant polyester according to claim 1, **characterized in that** the extraction in the a2 is microwave extraction, an extraction temperature is 35°C - 160°C, and an extraction duration is 10 min - 120 min.

**10.** The recovery method for a flame-retardant polyester according to claim 1, **characterized in that** an average particle size of the polyester A after the crushing treatment in the S1 is 2 mm - 10 mm; and an average particle size of the polyester B after the crushing treatment in the a1 is 0.02 mm - 1 mm.

**Patentansprüche**

**1.** Rückgewinnungsverfahren für einen flammhemmenden Polyester, **dadurch gekennzeichnet, dass**

es bestimmt wird, dass der flammhemmende Polyester ein Polyester A oder ein Polyester B gemäß einem Typ eines Flammschutzmittels ist; der Polyester A ein Polyester ist, in dem das Flammschutzmittel eines oder mehrere von bromiertem Polystyrol, Polydibromstyrol, Decabromdiphenylethan, Decabromdiphenylether, Hexabromcyclododecan und bromiertem Epoxid ist; der Polyester B ein Polyester ist, in dem das Flammschutzmittel eines oder mehrere von einem Phosphat-Flammschutzmittel, Tetrabrombisphenol A, Tetrabrombisphenol S und bromiertem Polycarbonat ist;
wenn der flammhemmende Polyester der Polyester A ist, das Verfahren die folgenden Schritte umfasst:

S1, Unterwerfen des Polyesters A einer Zerkleinerungsbehandlung;
S2, Unterwerfen des Polyesters A, der im S1 zerkleinert wurde, einer Mikrowellenalkoholyse und Durchführen von Trennung und Filtration, um ein Filtrat X und einen Filterrückstand a zu erhalten;
S3, Unterwerfen des Filtrats X im S2 einer Mikrowellenalkoholyse, dann Durchführen von Trennung und Reinigung, um ein Monomer oder ein Oligomer und ein Filtrat Y zu erhalten, und Reinigen des Filtrats Y, um einen Alkoholstoff zurückzugewinnen;
S4, Unterwerfen des Filterrückstands a im S2 einer Extraktion mit einem gezielten Extraktionsmittel, und Durchführen von Trennung und Reinigung, um das Flammschutzmittel und einen unlöslichen Stoff M zu erhalten;
worin ein Alkoholysemittel, das für die Mikrowellenalkoholyse im S2 und im S3 benutzt wird, Ethylenglykol oder Butandiol ist; das gezielte Extraktionsmittel im S4 zumindest eines von Methanol, Ethanol, Isopropanol, Azeton, Chloroform, Tetrahydrofuran, Toluol und Limonen ist;
wenn der flammhemmende Polyester der Polyester B ist, das Verfahren die folgenden Schritte umfasst:

a1, Unterwerfen des Polyesters B einer Zerkleinerungsbehandlung;
a2, Unterwerfen des Polyesters B, der im a1 zerkleinert wurde, einer Extraktion mit einem gezielten Extraktionsmittel und Durchführen von Trennung und Reinigung, um das Flammschutzmittel und einen unlöslichen Stoff N zu erhalten;
a3, Unterwerfen des unlöslichen Stoffes N im a2 einer Mikrowellenalkoholyse, und Durchführen von Trennung und Filtration, um ein Filtrat E und einen Filterrückstand b zu erhalten;
a4, Unterwerfen des Filtrats E im a3 einer Mikrowellenalkoholyse, dann Durchführen von Trennung und Reinigung, um ein Monomer oder ein Oligomer und ein Filtrat F zu erhalten, und Reinigen des Filtrats F, um einen Alkoholstoff zurückzugewinnen;

worin das gezielte Extraktionsmittel im a2 zumindest eines von Methanol, Ethanol, Isopropanol, Azeton, Chloroform, Tetrahydrofuran, Toluol und Limonen ist; und ein Alkoholysemittel, das für die Mikrowellenalkoholyse im a3 und im a4 benutzt wird, Ethylenglykol oder Butandiol ist.

2. Rückgewinnungsverfahren für einen flammhemmenden Polyester nach Anspruch 1, **dadurch gekennzeichnet, dass**, wenn das Alkoholysemittel das Butandiol ist, eine Temperatur der Mikrowellenalkoholyse im S2 und im S3 220°C - 230°C ist, und eine Dauer der Mikrowellenalkoholyse 10 Min. - 30 Min. ist.

3. Rückgewinnungsverfahren für einen flammhemmenden Polyester nach Anspruch 1, **dadurch gekennzeichnet, dass**, wenn das Alkoholysemittel das Ethylenglykol ist, eine Temperatur der Mikrowellenalkoholyse im a3 und im a4 200°C - 210°C ist, und eine Dauer der Mikrowellenalkoholyse 5 Min. - 10 Min. ist.

4. Rückgewinnungsverfahren für einen flammhemmenden Polyester nach Anspruch 1, **dadurch gekennzeichnet, dass** der unlösliche Stoff M mit wässrigem Ammoniak gelöst ist, um Antimontrioxid zu trennen und zurückzugewinnen.

5. Rückgewinnungsverfahren für einen flammhemmenden Polyester nach Anspruch 1, **dadurch gekennzeichnet, dass** ein Katalysator, der für die Mikrowellenalkoholyse im S2 und im S3 benutzt wird, eines bzw. eine oder mehrere von Zinkacetat, Butyltitanat, einer Titanatnanoröhre, Titanglykolat, einer ionischen Flüssigkeit aus 1,3-Dimethylimidazoltetrachlorferrat, einer ionischen Flüssigkeit aus 1-Allyl-3-Methylimidazol und Eisenchlorid ist.

6. Rückgewinnungsverfahren für einen flammhemmenden Polyester nach Anspruch 1, **dadurch gekennzeichnet, dass** ein Katalysator, der für die Mikrowellenalkoholyse im a3 und im a4 benutzt wird, eines bzw. eine oder mehrere von Zinkacetat, Butyltitanat, einer Titanatnanoröhre, Titanglykolat, einer ionischen Flüssigkeit aus 1,3-Dimethylimidazoltetrachlorferrat, einer ionischen Flüssigkeit aus 1-Allyl-3-Methylimidazol und Eisenchlorid ist.

7. Rückgewinnungsverfahren für einen flammhemmenden Polyester nach Anspruch 5 oder 6, **dadurch gekennzeichnet, dass** eine Zusatzmenge des Katalysators 0.05% - 1% einer Masse des flammhemmenden Polyesters ist.

8. Rückgewinnungsverfahren für einen flammhemmenden Polyester nach Anspruch 1, **dadurch gekennzeichnet, dass** die Extraktion im S4 Mikrowellenextraktion ist, eine Extraktionstemperatur 35°C - 160°C ist, und eine Extraktionsdauer 10 Min. - 120 Min. ist.

9. Rückgewinnungsverfahren für einen flammhemmenden Polyester nach Anspruch 1, **dadurch gekennzeichnet, dass** die Extraktion im a2 Mikrowellenextraktion ist, eine Extraktionstemperatur 35°C - 160°C, und eine Extraktionsdauer 10 Min. - 120 Min. ist.

10. Rückgewinnungsverfahren für einen flammhemmenden Polyester nach Anspruch 1, **dadurch gekennzeichnet, dass** eine durchschnittliche Teilchengröße des Polyesters A nach der Zerkleinerungsbehandlung im S1 2 mm - 10 mm ist; und eine durchschnittliche Teilchengröße des Polyesters B nach der Zerkleinerungsbehandlung im a1 0.02 mm - 1 mm ist.

**Revendications**

1. Procédé de récupération d'un polyester ignifuge, **caractérisé en ce que**

le polyester ignifuge est déterminé comme étant un polyester A ou un polyester B selon un type de retardateur de flamme ; le polyester A est un polyester dans lequel le retardateur de flamme est un ou plusieurs parmi le polystyrène bromé, le polydibromostyrène, le décabromodiphényléthane, le décabromodiphényléther, l'hexabromocyclododécane et l'époxy bromé ; le polyester B est un polyester dans lequel le retardateur de flamme est un ou plusieurs parmi un retardateur de flamme au phosphate, le tétrabromobisphénol A, le tétrabromobisphénol S et le polycarbonate bromé ;

lorsque le polyester ignifuge est le polyester A, le procédé comprend les étapes suivantes :

S1, soumettre le polyester A à un traitement de broyage ;
S2, soumettre le polyester A broyé dans le S1 à une alcoolyse aux micro-ondes, et effectuer une séparation et une filtration pour obtenir un filtrat X et un résidu de filtre a ;

S3, soumettre le filtrat X dans le S2 à une alcoolyse aux micro-ondes, puis effectuer une séparation et une purification pour obtenir un monomère ou un oligomère et un filtrat Y, et purifier le filtrat Y pour récupérer une substance alcoolique ;

S4, soumettre le résidu de filtre a dans le S2 à une extraction avec un extractant ciblé, et effectuer une séparation et une purification pour obtenir le retardateur de flamme et une substance insoluble M ;

dans lequel un agent d'alcoolyse utilisé pour l'alcoolyse par micro-ondes dans le S2 et le S3 est l'éthylène glycol ou le butanediol ; l'agent d'extraction ciblé dans le S4 est au moins l'un parmi le méthanol, l'éthanol, l'isopropanol, l'acétone, le chloroforme, le tétrahydrofurane, le toluène et le limonène ;

lorsque le polyester ignifuge est le polyester B, le procédé comprend les étapes suivantes :

a1, soumettre le polyester B à un traitement de broyage ;

a2, soumettre le polyester B broyé dans l'a1 à une extraction avec un extractant ciblé, et effectuer une séparation et une purification pour obtenir le retardateur de flamme et une substance insoluble N ;

a3, soumettre la substance insoluble N dans l'a2 à une alcoolyse aux micro-ondes, et effectuer une séparation et une filtration pour obtenir un filtrat E et un résidu de filtre b ;

a4, soumettre le filtrat E dans l'a3 à une alcoolyse aux micro-ondes, puis effectuer une séparation et une purification pour obtenir un monomère ou un oligomère et un filtrat F, et purifier le filtrat F pour récupérer une substance alcoolique ;

dans lequel l'agent d'extraction ciblé dans l'a2 est au moins l'un parmi le méthanol, l'éthanol, l'isopropanol, l'acétone, le chloroforme, le tétrahydrofurane, le toluène et le limonène ; et un agent d'alcoolyse utilisé pour l'alcoolyse par micro-ondes dans l'a3 et l'a4 est l'éthylène glycol ou le butanediol.

**2.** Procédé de récupération d'un polyester ignifuge selon la revendication 1, **caractérisé en ce que** lorsque l'agent d'alcoololyse est le butanediol, une température de l'alcoolyse par micro-ondes dans le S2 et le S3 est de 220°C à 230°C, et une durée de l'alcoolyse par micro-ondes est de 10 min à 30 min.

**3.** Procédé de récupération d'un polyester ignifuge selon la revendication 1, **caractérisé en ce que** lorsque l'agent d'alcoolyse est l'éthylène glycol, une température de l'alcoolyse par micro-ondes dans l'a3 et l'a4 est de 200°C à 210°C, et une durée de l'alcoolyse par micro-ondes est de 5 min à 10 min.

**4.** Procédé de récupération d'un polyester ignifuge selon la revendication 1, **caractérisé en ce que** la substance insoluble M est dissoute avec de l'ammoniaque pour séparer et récupérer le trioxyde d'antimoine.

**5.** Procédé de récupération d'un polyester ignifuge selon la revendication 1, **caractérisé en ce qu'**un catalyseur utilisé pour l'alcoolyse par micro-ondes dans le S2 et le S3 est un ou plusieurs éléments parmi l'acétate de zinc, le titanate de butyle, un nanotube de titanate, le glycolate de titane, un liquide ionique de tétrachloroferrate de 1,3-diméthylimidazolium, un liquide ionique de 1-allyl-3-méthylimidazolium et le chlorure ferrique.

**6.** Procédé de récupération d'un polyester ignifuge selon la revendication 1, **caractérisé en ce qu'**un catalyseur utilisé pour l'alcoolyse par micro-ondes dans le a3 et le a4 est un ou plusieurs éléments parmi l'acétate de zinc, le titanate de butyle, un nanotube de titanate, le glycolate de titane, un liquide ionique de tétrachloroferrate de 1,3-diméthylimidazolium, un liquide ionique de 1-allyl-3-méthylimidazolium et le chlorure ferrique.

**7.** Procédé de récupération d'un polyester ignifuge selon la revendication 5 ou 6, **caractérisé en ce qu'**une quantité d'addition du catalyseur est de 0.05% à 1% d'une masse du polyester ignifuge.

**8.** Procédé de récupération d'un polyester ignifuge selon la revendication 1, **caractérisé en ce que** l'extraction dans le S4 est une extraction par micro-ondes, une température d'extraction de 35°C à 160°C et une durée d'extraction de 10 min à 120 min.

**9.** Procédé de récupération d'un polyester ignifuge selon la revendication 1, **caractérisé en ce que** l'extraction dans le a2 est une extraction par micro-ondes, une température d'extraction de 35°C à 160°C et une durée d'extraction de 10 min à 120 min.

**10.** Procédé de récupération d'un polyester ignifuge selon la revendication 1, **caractérisé en ce qu'**une taille moyenne de particules du polyester A après le traitement de broyage dans le S1 est de 2 mm à 10 mm ; et une taille moyenne de particules du polyester B après le traitement de broyage dans le a1 est de 0.02 mm à 1 mm.

FIG. 1

FIG. 2

EP 4 592 339 B1

FIG. 3

FIG. 4

## REFERENCES CITED IN THE DESCRIPTION

**Patent documents cited in the description**

- WO 2022033962 A1
- WO 2019174656 A1